# FASCICULE DE BREVET EUROPEEN

(11) **EP 4 178 475 B1**
(45) Date de publication et mention de la délivrance du brevet: **23.04.2025**
(21) Numéro de dépôt: 21734412.6
(22) Date de dépôt: 28.05.2021
(51) Int. Cl.: A61B 18/14, A61B 18/00

(54) **PINCE ELECTRO-CHIRURGICALE COMPRENANT UN DISPOSITIF D'ASPIRATION**
ELEKTROCHIRURGISCHE ZANGE MIT ABSAUGVORRICHTUNG
ELECTROSURGICAL FORCEPS COMPRISING A SUCTION DEVICE

(30) Priorité: 08.07.2020 FR 2007202
(43) Date de publication de la demande: 17.05.2023
(73) Titulaire: Magnitude Surgical, 33410 Omet (FR)
(72) Inventeur: ROUSSEAU, Nicolas, 33410 Omet (FR)
(74) Mandataire: IP Trust
(86) Numéro de dépôt international: PCT/FR2021/050972
(87) Numéro de publication internationale: WO 2022/008808

(56) Documents cités:
- EP-A1- 2 153 791
- WO-A1-2015/193627
- US-A1- 2003 139 743
- US-A1- 2007 255 272
- US-A1- 2010 087 814
- US-A1- 2011 306 967
- US-A1- 2017 319 264
- US-B1- 6 926 717

## Description

### DOMAINE DE L'INVENTION

La présente invention concerne une pince électro-chirurgicale comprenant un dispositif d'aspiration des fumées susceptibles d'être émises au cours d'une intervention. L'invention s'applique plus particulièrement à une pince chirurgicale bipolaire comprenant deux électrodes disposées aux extrémités respectives de deux membres articulés.

### ARRIERE PLAN TECHNOLOGIQUE DE L'INVENTION

Les dispositifs électro-chirurgicaux, mono polaires ou bipolaires, sont très utilisés dans le milieu chirurgical pour inciser, manipuler et/ou cautériser des tissus. Toutefois, les fumées émises par ces instruments au cours de leur utilisation sont susceptibles de contenir des éléments chimiques pouvant, si inhalés, nuire à la santé du patient ainsi qu'à celle du chirurgien. Ces fumées peuvent notamment contenir des substances irritantes et/ou cancérigènes. Il est donc souhaitable de limiter le risque de contamination en disposant de pinces chirurgicales capable d'aspirer la fumée générée pour éviter son inhalation.

On connait une telle pince du document US20170319264. L'extrémité proximale de cette pince est reliée à un dispositif d'aspiration et les parois internes des membres de la pince sont configurées pour former, en combinaison, un conduit d'aspiration lorsque ces deux membres sont plaqués l'une contre l'autre pour cautériser des tissus.

Le document WO2015193627 divulgue une pince électro-chirurgicale polyvalente pouvant fonctionner en préhension, incision et en cautérisation. Cette pince peut traiter des tissus dans ces différents modes de fonctionnement sans nécessairement ajuster le niveau d'énergie et/ou la forme d'onde délivrés par le générateur pendant l'intervention. Une première électrode, disposée à l'extrémité distale d'un premier membre, présente une section transversale concave, apte à loger au moins en partie une seconde électrode. La seconde électrode, disposée à l'extrémité distale d'un second membre articulé au premier, est en saillie de la première électrode lorsque les deux membres sont rapprochés l'un de l'autre. Comme cela est précisé dans le détail dans le document précité, la pince peut être utilisée selon plusieurs modes de fonctionnement différents. Dans chacun de ces modes, une portion particulière des électrodes est mise en contact avec les tissus. En conséquence, l'origine précise des fumées générées au cours de l'utilisation de cette pince est variable selon le mode de fonctionnement exploité. Les documents US 2003/139743 A1, US 6 926 717 B1, US 2010/087814 A1 et US 2007/255272 A1 divulguent d'autres pinces électro-chirurgicales munies d'un dispositif d'aspiration.

Il serait souhaitable de pouvoir disposer d'une pince électro-chirurgicale munie d'un dispositif d'aspiration permettant une aspiration efficace des fumées indépendamment du mode de fonctionnement choisi.

### OBJET DE L'INVENTION

Un but de l'invention est de proposer une telle pince électro-chirurgicale comprenant un dispositif d'aspiration de fumée. Un autre but de l'invention est de fournir un dispositif d'aspiration de fumée qui puisse équiper une pince électro-chirurgicale existante.

### BREVE DESCRIPTION DE L'INVENTION

En vue de la réalisation de ce but, l'objet de l'invention propose une pince électro-chirurgicale selon la revendication 1.

Selon d'autres caractéristiques avantageuses et non limitatives de l'invention, prises seules ou selon toute combinaison techniquement réalisable :
- la gouttière d'aspiration est formée de deux parois latérales et d'une base, la base comprenant un bec pour recevoir la première électrode et prévenir un flux d'aspiration au pied de cette première électrode ;
- les parois comprennent, sur leur surface interne, des éléments de guidage pour orienter la fumée vers le conduit d'aspiration ;
- la deuxième électrode présente une forme de lame présentant une surface plane, et l'élément obturateur comprend deux méplats disposés parallèlement à la surface plane ;
- la deuxième électrode comprend un manipulateur pour déplacer en rotation la seconde électrode sur elle-même ;
- le premier membre comprend également un canal d'irrigation débouchant sur une buse d'irrigation disposée directement sous la première électrode ;
- le corps longitudinal est clippé au premier membre à l'aide d'une paire de crans disposés sur une partie proximale du corps longitudinal ;

### BREVE DESCRIPTION DES FIGURES

D'autres caractéristiques et avantages de l'invention ressortiront de la description détaillée de l'invention qui va suivre en référence aux figures annexées sur lesquels :
[Fig. 1] La figure 1 représente une vue d'ensemble d'une pince électro-chirurgicale conforme à l'invention ;
[Fig. 2] La figure 2 représente une coupe du corps longitudinal d'un dispositif d'aspiration conforme à l'invention ;
[Fig. 3] La figure 3 représente une vue en perspective d'une gouttière du dispositif d'aspiration de la figure 2 ;
[Fig. 4]

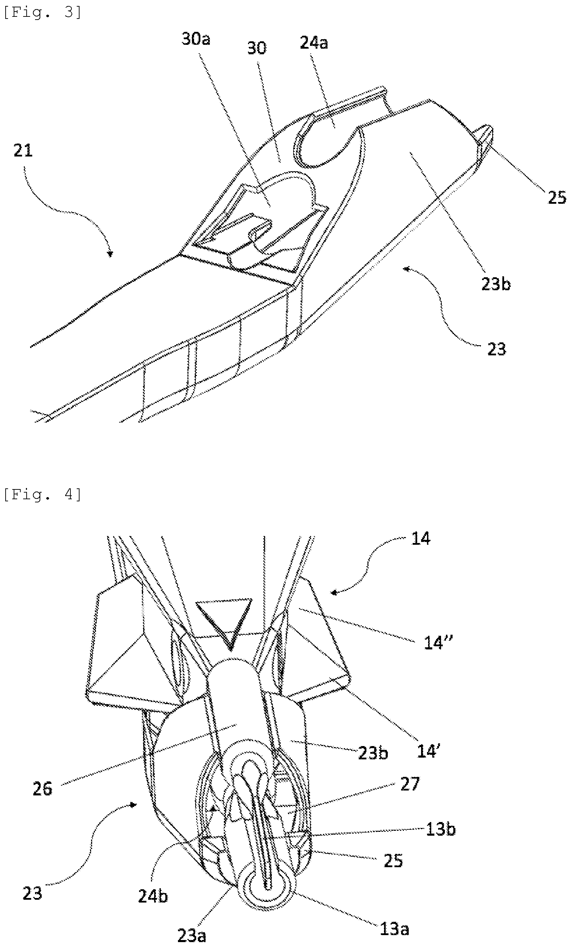

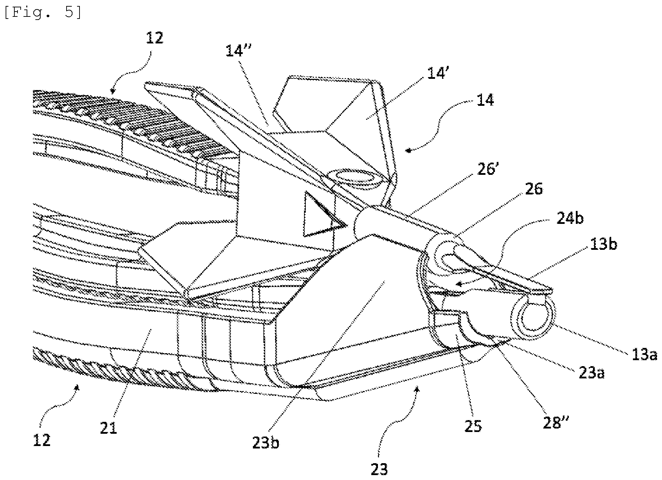

   [Fig. 5] Les figures 4 et 5 représentent l'extrémité distale d'une pince conforme à l'invention, dans deux modes de fonctionnement différents.

### DESCRIPTION DETAILLEE DE L'INVENTION

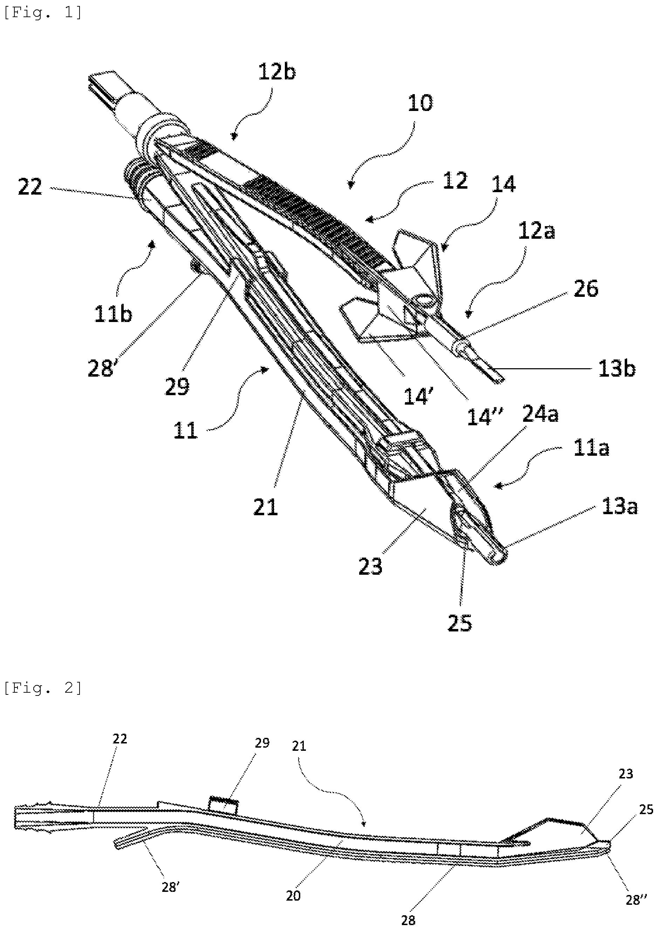

En référence à la figure 1, la pince 10 comprend un premier membre 11 et un second membre 12 reliés par un pivot d'articulation permettant de rapprocher et d'éloigner les membres 11,12 l'un de l'autre. Le pivot d'articulation est ici positionné à l'extrémité proximale de chaque membre, mais il pourrait tout aussi bien être placé dans la partie centrale des membres 11,12 pour former des ciseaux. La pince 10 est électriquement reliée à un générateur (non représenté sur les figures) apte à générer les signaux hautes fréquences conduisant à la réalisation des traitements, tels qu'une incision et/ou une cautérisation. Il peut s'agir d'un générateur de courant.

La pince 10 comprend également une première électrode 13a et une deuxième électrode 13b respectivement disposées aux extrémités distales 11a,12a du premier membre 11 et du deuxième membre 12. Les électrodes 13a, 13b sont en liaison électrique avec le générateur, par exemple par l'intermédiaire d'un câble intégré aux membres 11, 12 pour former un premier et deuxième contact électrique avec les tissus biologiques, et refermer des lignes de courant sur ces tissus.

La première électrode 13a présente une section transversale concave, apte à loger en partie la seconde électrode 13b, ici en forme de lame présentant une surface plane et une tranche qui peuvent l'une et l'autre être exploitées au cours d'un traitement. La seconde électrode 13b est en saillie de la première électrode 13a lorsque les deux membres 11,12 sont en position rapprochées l'un de l'autre.

Dans l'exemple représenté, la pince électro-chirurgicale est munie d'un dispositif d'aspiration. Celui-ci est formé d'un corps longitudinal 21 assemblé au premier membre 11, représenté en coupe sur la figure 2, et d'un élément obturateur 26 disposé sur le second membre 12.

Le corps longitudinal 21 présente, à une première extrémité, un raccord 22 pour relier un canal 20 à un conduit d'aspiration d'un équipement externe. Le canal 20 est formé, à sa deuxième extrémité opposée à la première extrémité, d'une gouttière d'aspiration 23 destinée à recevoir l'extrémité distale 11a du premier membre 11. La gouttière d'aspiration 23 présente une ouverture supérieure 24a, que l'on appellera par la suite ouverture principale 24a, et une ouverture axiale 24b, qui sont exploitées toutes les deux pour évacuer les fumées comme cela sera détaillé dans la suite de cet exposé. Lorsque le corps longitudinal 21 est assemblé convenablement à la pince, une partie au moins de la première électrode 13a est saillante de la gouttière d'aspiration 23.

Comme cela est visible sur la figure 2, le canal 20 est un canal fermé qui est enfoui sur une partie de sa longueur dans le corps longitudinal 21. Cette portion fermée du canal débouche d'un côté sur la gouttière d'aspiration 23 et de l'autre sur le raccord 22. Le canal 20 permet d'évacuer la fumée formée à l'extrémité distale de la pince 10, dans l'environnement proche des électrodes 13a, 13b et captée par l'une au moins des ouvertures 24a,24b de la gouttière d'aspiration 23, vers le conduit d'aspiration. La gouttière 23 forme donc une buse d'aspiration de ces fumées, et sa configuration permet d'aspirer efficacement les fumées dans les multiples modes de fonctionnement de la pince.

La figure 3 présente une vue en perspective de la gouttière d'aspiration 23, sur laquelle est bien visible l'ouverture principale 24a de la gouttière 23. Un capot arrière 30 est muni d'un passage 30a conformé au contour de l'extrémité distale 11a du premier membre 11 de la pince. La pince 10 est assemblée au corps longitudinal 21 en introduisant l'extrémité distale 11a du premier membre 11, portant la première électrode 13a, par le passage 30a. En position d'assemblage, le passage 30a est entièrement obturé par le premier membre 11 de la pince, ce qui limite ou élimine tout flux d'aspiration parasite au niveau de ce passage 30a lorsque le mécanisme d'aspiration de la pince est activé. Pour retenir fixement le corps longitudinal 21 sur le premier membre 11, le corps longitudinal 21 est muni d'un élément de retenu 29, ici une paire de crans 29 disposés sur une partie proximale, pour se clipper sur le premier membre 11.

Comme cela est visible sur les figures 4 et 5, la gouttière d'aspiration 23 comprend une base 23a se terminant par un bec 25 présentant un évidemment permettant de recevoir et de porter la première électrode 13a. La gouttière d'aspiration 23 comprend également deux parois 23b latérales définissant les ouvertures principale et axiale. Le canal d'aspiration 20 débouche dans la base 23a de la gouttière 23 comme cela est bien visible sur la figure 2. La gouttière présente une dimension telle qu'un espacement latéral existe entre le premier membre 11, la première électrode 13a et les parois 23b. Cet espacement qui peut être compris entre 1 mm et 5 mm de chaque côté de la première électrode 13a définit l'ouverture axiale 24b permettant une aspiration au plus proche de la première électrode 13a comme cela est bien visible sur la figure 4. Similairement, la distance séparant les deux parois 23b à leurs extrémités libres définit l'ouverture principale 24a de la gouttière 23.

Les parties latérales du bec 25, de part et d'autre de l'évidemment recevant la première électrode 13a, permettent de prévenir une aspiration trop proche de cette électrode 13a. On évite ainsi d'aspirer des fluides ou d'autres éléments non souhaités qui pourraient bloquer et/ou endommager le canal d'aspiration 20. Comme cela est visible sur la figure 2, les parties latérales du bec 25, à l'intérieur de la gouttière 23, sont conformées en rampes, pour orienter convenablement le flux d'aspiration axial.

De manière avantageuse, les parois 23b peuvent comprendre sur leurs surfaces internes, orientées vers l'intérieur de la gouttière d'aspiration 23, des éléments de guidage 27 pour orienter la fumée aspirée vers l'ouverture le canal d'aspiration 20.

Le deuxième membre 12 de la pince est configuré pour refermer au moins partiellement l'ouverture principale 24a de la gouttière d'aspiration 23 lorsque les deux membres 11, 12 sont en position rapprochée l'un de l'autre. A cet effet, le deuxième membre 12 est muni de l'élément obturateur 26. Celui-ci est disposé le long de ce membre 12 pour être en vis-à-vis de l'ouverture principale 24a de la gouttière 23 et permettre de se positionner entre les deux parois latérales 23b en rapprochant les deux membres 11, 12. L'élément obturateur 26 se présente ici sous la forme d'une pièce sensiblement cylindrique, emmanchée sur le second membre 12. En refermant la gouttière d'aspiration à l'aide de l'élément obturateur 26, même partiellement, on renforce les flux d'aspiration au niveau de l'ouverture axiale de la gouttière et on améliore l'évacuation des fumées produites dans l'environnement directe de cette ouverture.

Revenant à la description de la figure 1, le deuxième membre 12 de la pince est avantageusement muni d'un manipulateur 14 permettant de déplacer en rotation la seconde électrode 13b sur elle-même (autour d'un axe confondu avec cette électrode) et de positionner cette dernière selon différentes orientations par rapport à la première électrode 13a. Le manipulateur 14 prend ici la forme d'un élément en rosace 14 présentant quatre ailes 14' définissant quatre encoches 14" permettant de recevoir le premier membre 11 lorsque les deux membres 11,12 sont rapprochés l'un de l'autre. L'élément en rosace 14 est libre de se mouvoir en rotation autour d'un axe sensiblement défini par l'axe longitudinal du deuxième membre 12, et entraîne en rotation la seconde électrode 13b et l'élément obturateur 26.

Les deux électrodes 13a, 13b (lorsque le second membre est rapproché du premier) dépassent de la gouttière d'aspiration 23 de sorte que cette gouttière (et plus généralement le dispositif d'aspiration) ne gêne pas l'utilisation de la pince au cours d'une intervention. L'ouverture principale 24a de la gouttière d'aspiration 23 autorise notamment de rapprocher librement le deuxième membre du premier, et de positionner la seconde électrode 13b à proximité de la première électrode 13a (figure 5) ou de la loger dans la concavité de cette première électrode (figure 4), permettant un fonctionnement sans entrave de la pince électro-chirurgicale dans tous ses modes de fonctionnement conformément à l'enseignement du document WO2015193627.

Lorsque les deux membres 11, 12 de la pince sont ouverts, dans une configuration permettant la préhension des tissus entre les deux électrodes 13a, 13b, l'ouverture principale 24a de la gouttière n'est pas refermée par l'élément obturateur 26, et des flux d'aspiration dit « radiaux » peuvent s'écouler par cette ouverture, au plus près des tissus, pour évacuer les éventuelles fumées qui seraient émises.

Dans un autre mode de fonctionnement représenté sur la figure 5 la seconde électrode 13b en forme de lame est orientée et positionnée à l'aide de l'élément en rosace 14 pour que sa surface plane surplombe la première électrode 13a concave. Dans cette configuration l'élément obturateur 26 obstrue pleinement l'ouverture 24a de la gouttière 23, et vient au plus proche des parois 23b de la gouttière 23, par exemple à moins de 0,2mm de ces parois. On limite ainsi ou on empêche la présence d'un flux radial par l'ouverture principale 24a, pour privilégier le flux d'aspiration axial, permettant d'aspirer les fumées produites à l'extrémité de la surface plane de la deuxième électrode 13b, par exemple lorsque celle-ci travaille en cautérisation.

Dans encore un autre mode de fonctionnement représenté sur la figure 4, la seconde électrode 13b en forme de lame est orientée et positionnée à l'aide de l'élément en rosace 14 pour être logée partiellement dans la concavité de la première électrode 13a. Dans cette configuration, le second membre 12 est disposé au plus proche du premier membre 11, et l'élément obturateur 26 pénètre un peu plus profondément entre les deux parois 23b, et s'engage un peu plus profondément dans la gouttière 23. L'élément obturateur 26 présentant une forme cylindrique, cet engagement libère un jeu entre l'élément obturateur 26 et chacune des parois 23b. Ce jeu, qui peut être de l'ordre du millimètre, permet de développer un flux d'aspiration radial permettant d'aspirer les fumées produites à proximité du tranchant de la deuxième électrode 13b, par exemple lorsque celle-ci travaille en incision. Bien entendu, l'élément obturateur 26 peut présenter une autre forme que celle, cylindrique, représentée sur les figures, toute forme faisant varier sa dimension transversale selon son degré d'engagement dans la gouttière 23 permettant d'obtenir le même effet.

Dans un mode de mise en œuvre particulier, optionnel mais très avantageux pour favoriser le développement du flux d'aspiration radial dans la configuration qui vient d'être décrite, l'élément obturateur 26 de forme cylindrique peut être muni sur sa surface externe de deux méplats 26' diamétralement opposés. Les surfaces de ces méplats 26' sont parallèles à la surface plane de la seconde électrode 13b. Les méplats 26' permettent de réduire la dimension transversale de l'élément obturateur 26 dans cette configuration. Ainsi, lorsque la seconde électrode 13b est orientée à l'aide de l'élément en rosace 14 pour pouvoir se loger dans la concavité de la première électrode 13a, les méplats 26' sont quant à eux orientés pour faire face aux parois 23b de la gouttière. L'élément obturateur 26 comble alors partiellement l'ouverture 24a de cette gouttière 23, ce qui favorise le développement du flux d'aspiration radial.

En plus d'un canal d'aspiration 20, le corps longitudinal 21 peut être muni d'un canal d'irrigation 28, visible sur la figure 2. Le canal d'irrigation 28 est avantageusement disposé sensiblement parallèle au canal d'aspiration 20. Ce canal d'irrigation 28 est également fermé et intégré au corps longitudinal 21. Il débouche sur un raccord secondaire 28' pour être relié à un conduit d'irrigation d'un équipement, configuré pour dispenser un liquide dans ce canal. De l'autre côté du corps longitudinal 21, le canal d'irrigation 28 débouche sur une buse d'irrigation 28". La buse d'irrigation 28" débouche au niveau du bec 26 de la base 23a de la gouttière d'aspiration 23, directement sous la première électrode 13a, c'est-à-dire au contact de cette électrode. De la sorte, le fluide circulant dans le conduit d'irrigation 28 peut perler et/ou ruisseler le long de cette électrode 13a pour atteindre les tissus, quelle que soit l'orientation de la pince et des électrodes. Dans une configuration alternative et non représentée sur les figures, la buse d'irrigation 28" ne débouche pas directement sous la première électrode 13a. On peut prévoir dans ce cas que le canal d'irrigation présente, du côté de la première électrode, d'un coude permettant de faire déboucher le canal à distance de la première électrode. En éloignant ainsi la buse d'irrigation de la première électrode, on limite le risque d'aspirer le liquide qui se déverse de la buse dans le canal d'aspiration.

Bien entendu l'invention n'est pas limitée au mode de mise en œuvre décrit et on peut y apporter des variantes de réalisation sans sortir du cadre de l'invention tel que défini par les revendications.

Le dispositif d'aspiration (et notamment le corps longitudinal) a été présenté et décrit comme un élément séparé et assemblable à la pince électro-chirurgicale, ce qui forme un avantage important de la présente invention.

Les électrodes peuvent être de formes variées et non uniquement restreintes aux formes illustrées.

## Revendications

1. Pince électro-chirurgicale (10) comprenant :
- un premier membre (11) et un second membre (12) reliés par un pivot d'articulation permettant de rapprocher et d'éloigner les membres (11,12) l'un de l'autre, les deux membres (11,12) présentant une extrémité distale (11a,12a) et une extrémité proximale (11b, 12b);
- une première électrode (13a) et une deuxième électrode (13b) respectivement disposées aux extrémités distales (11a,12a) du premier membre (11) et du deuxième membre (12);
- un corps longitudinal (21) assemblé au premier membre (11) et dans lequel est disposé un canal (20), le corps principal (21) se terminant, du côté de l'extrémité distale (11a) du premier membre (11), par une gouttière d'aspiration (23) présentant une ouverture supérieure (24a) et une ouverture axiale (24b),
- un élément obturateur (26) disposé sur le second membre (12) et configuré pour combler au moins en partie l'ouverture supérieure (24a) de la gouttière d'aspiration (23) lorsque les deux membres (11,12) sont dans une position rapprochée l'un de l'autre.

2. Pince électro-chirurgicale (10) selon la revendication précédente dans lequel la gouttière d'aspiration (23) est formée de deux parois latérales (23b) et d'une base (23b), la base (23a) comprenant un bec (25) pour recevoir la première électrode (13a) et prévenir un flux d'aspiration au pied de cette première électrode (13a).

3. Pince électro-chirurgicale (10) selon la revendication précédente dans lequel les parois (23b) comprennent, sur leur surface interne, des éléments de guidage (27) pour orienter la fumée vers le conduit d'aspiration (20).

4. Pince électro-chirurgicale (10) selon la revendication 1 dans lequel la deuxième électrode (13b) présente une forme de lame présentant une surface plane, et l'élément obturateur (26) comprend deux méplats (26') disposés parallèlement à la surface plane.

5. Pince électro-chirurgicale (10) selon l'une des revendications précédentes dans lequel la deuxième électrode (13b) comprend un manipulateur (14) pour déplacer en rotation la seconde électrode (13b) sur elle-même.

6. Pince électro-chirurgicale (10) selon l'une des revendications précédentes dans lequel le premier membre (11) comprend également un canal d'irrigation (28) débouchant sur une buse d'irrigation (28") disposée directement sous la première électrode (13a).

7. Pince électro-chirurgicale (10) selon l'une des revendications précédentes dans lequel le corps longitudinal (21) est clippé au premier membre (11) à l'aide d'une paire de crans (29) disposés sur une partie proximale du corps longitudinal (21).

## Patentansprüche

1. Elektrochirurgische Zange (10), umfassend:
- ein erstes Element (11) und ein zweites Element (12), die durch einen Gelenkzapfen verbunden sind, der es ermöglicht, die Elemente (11, 12) aneinander anzunähern und voneinander zu entfernen, wobei die zwei Elemente (11, 12) ein distales Ende (11a, 12a) und ein proximales Ende (11b, 12b) aufweisen;
- eine erste Elektrode (13a) und eine zweite Elektrode (13b), die jeweils an den distalen Enden (11a, 12a) des ersten Elements (11) und des zweiten Elements (12) angeordnet sind;
- einen Längskörper (21), der an dem ersten Element (11) montiert ist und in dem ein Kanal (20) angeordnet ist, wobei der Hauptkörper (21) auf der Seite des distalen Endes (11a) des ersten Elements (11) durch eine Saugrinne (23) endet, die eine obere Öffnung (24a) und eine axiale Öffnung (24b) aufweist,
- ein Verschlusselement (26), das an dem zweiten Element (12) angeordnet und zum Ausfüllen mindestens teilweise der oberen Öffnung (24a) der Saugrinne (23) konfiguriert ist, wenn die zwei Elemente (11, 12) in einer aneinander angenäherten Position sind.

2. Elektrochirurgische Zange (10) nach dem vorstehenden Anspruch, wobei die Saugrinne (23) aus zwei Seitenwänden (23b) und einer Basis (23b) ausgebildet ist, die Basis (23a) umfassend eine Backe (25) zum Aufnehmen der ersten Elektrode (13a) und Verhindern eines Saugstroms an dem Fuß dieser ersten Elektrode (13a).

3. Elektrochirurgische Zange (10) nach dem vorstehenden Anspruch, wobei die Wände (23b) an ihrer Innenoberfläche Führungselemente (27) zum Lenken des Rauchs in Richtung der Saugleitung (20) aufweisen.

4. Elektrochirurgische Zange (10) nach Anspruch 1, wobei die zweite Elektrode (13b) eine Klingenform aufweist, die eine flache Oberfläche aufweist, und das Verschlusselement (26) zwei Abflachungen (26') umfasst, die parallel zu der flachen Oberfläche angeordnet sind.

5. Elektrochirurgische Zange (10) nach einem der vorstehenden Ansprüche, wobei die zweite Elektrode (13b) einen Manipulator (14) für eine Drehbewegung der zweiten Elektrode (13b) um sich selbst umfasst.

6. Elektrochirurgische Zange (10) nach einem der vorstehenden Ansprüche, wobei das erste Element (11) auch einen Spülkanal (28) umfasst, der in eine Spüldüse (28") mündet, die direkt unter der ersten Elektrode (13a) angeordnet ist.

7. Elektrochirurgische Zange (10) nach einem der vorstehenden Ansprüche, wobei der Längskörper (21) mit Hilfe eines Paars von Kerben (29), die an einem proximalen Abschnitt des Längskörpers (21) angeordnet sind, an dem ersten Element (11) eingeclipst ist.

## Claims

1. Electrosurgical forceps (10) comprising:
- a first arm (11) and a second arm (12) connected by a pivot joint for moving the arms (11,12) toward and away from one another, the two arms (11,12) having a distal end (11a,12a) and a proximal end (11b,12b);
- a first electrode (13a) and a second electrode (13b) arranged at the distal ends (11a, 12a) of the first arm (11) and the second arm (12), respectively;
- a longitudinal body (21) connected to the first arm (11) and in which a channel (20) is arranged, the main body (21) terminating, on the side of the distal end (11a) of the first arm (11), in a suction conduit (23) having an upper opening (24a) and an axial opening (24b),
- a shutter element (26) arranged on the second arm (12) and configured to at least partially fill up the upper opening (24a) of the suction conduit (23) when the two members (11,12) are in a position close to one another.

2. Electrosurgical forceps (10) according to the preceding claim, wherein the suction conduit (23) is formed by two side walls (23b) and a base (23b), the base (23a) comprising a spout (25) to receive the first electrode (13a) and to prevent a suction flow at the foot of this first electrode (13a).

3. Electrosurgical forceps (10) according to the preceding claim, wherein the walls (23b) comprise, on their internal surface, guide elements (27) to direct the smoke towards the suction duct (20).

4. Electrosurgical forceps (10) according to claim 1, wherein the second electrode (13b) has the shape of a blade having a flat surface, and the shutter element (26) comprises two flats (26') arranged in parallel with the flat surface.

5. Electrosurgical forceps (10) according to any of the preceding claims, wherein the second electrode (13b) comprises a manipulator (14) for rotating the second electrode (13b) on itself.

6. Electrosurgical forceps (10) according to any of the preceding claims, wherein the first arm (11) also comprises an irrigation channel (28) opening onto an irrigation nozzle (28") arranged directly under the first electrode (13a).

7. Electrosurgical forceps (10) according to any of the preceding claims, wherein the longitudinal body (21) is clipped to the first arm (11) using a pair of notches (29) arranged on a proximal part of the longitudinal body (21).
